# FASCICULE DE BREVET EUROPEEN

(11) **EP 0 544 882 B1**
(45) Date de publication et mention de la délivrance du brevet: **10.09.1997**
(21) Numéro de dépôt: 92913356.9
(22) Date de dépôt: 25.06.1992
(51) Int. Cl.: A61B 17/28, A61B 17/02, A61M 25/01, A61B 17/068

(54) **DISPOSITIF ORIENTABLE POUR LA MANIPULATION DES INSTRUMENTS CHIRURGICAUX NOTAMMENT EN CHIRURGIE COELIOSCOPIQUE**
Schwenkbare Vorrichtung zur Bedienung von chirurgischen Instrumenten insbesondere während der Laparoskopischen Chirurgie
POSITIONING DEVICE FOR OPERATING CHIRURGICAL INSTRUMENTS, PARTICULARLY IN CELIOSCOPIC SURGERY

(30) Priorité: 25.06.1991 FR 9108263
(43) Date de publication de la demande: 09.06.1993
(73) Titulaire: Sgro, Jean-Claude, F-21000 Dijon (FR)
(72) Inventeur: Sgro, Jean-Claude, F-21000 Dijon (FR)
(74) Mandataire: Dupuis, François
(86) Numéro de dépôt international: FR9200576
(87) Numéro de publication internationale: WO9300048

(56) Documents cités:
- EP-A- 0 029 344
- WO-A-89/11827
- DE-A- 3 509 787
- DE-U- 8 535 164
- FR-A- 2 065 112
- GB-A- 2 138 687
- US-A- 3 162 214
- US-A- 4 226 228
- US-A- 4 239 036
- US-A- 4 688 555

## Description

Ce dispositif concerne plus particulièrement la chirurgie sous coelioscopie ou sous vidéo-coelioscopie, c'est-à-dire sans grandes incisions du corps. Cette chirurgie est réalisée généralement par des accès transparietaux de faibles diamètres.

Les instruments actuellement utilisés en chirurgie colioscopique sont généralement cylindriques et rectilignes, pour pouvoir être introduits dans des trocards étanches rectilignes. Cependant, de tels instruments ne comportent aucun système d'orientation. Les mouvements de préhension ou de section, par exemple, se font dans l'axe général de l'instrument sans que l'extrémité de celui-ci puisse être orientée dans un autre axe. Cette forme rectiligne est nécessaire pour les introduire dans des trocards droits n'acceptant aucune courbure.

On connaît également des instruments de forme courbée, mais fixes, pouvant être introduits dans des trocards souples. Toutefois, les trocards souples sont d'utilisation plus difficile.

Le brevet WO-8911827 enseigne une pince à biopie souple introduite dans le canal opérateur d'un bronchoscope souple. La courbure de la pince est réalisée au moyen d'un câble opposé à un ressort, qui a pour effet de ramener l'extrémité toujours à la même position. Il n'y a donc pas de possibilité de faire un blocage dans la position angulaire souhaitée. Cette pince trouve une application uniquement dans le cas de prélèvements, mais ne peut pas être utilisée en chirurgie.

On connait également des appareils pour la manipulation d'instruments chirurgicaux présentant plusieurs éléments cylindriques creux et articulés entre eux. Les éléments sont solidaires d'un corps présentant une poignée de préhension. En outre, ces éléments peuvent être assujettis à un système de câbles pour être orientés à volonté avec capacité de blocage temporaire dans la position angulaire désirée.

Cet état de la technique ressort de l'enseignement des brevets EP-A-29344, DE-U-8535164.4 et US-A-4 239 036.

Le brevet EP-A-29344 concerne un dispositif orientable pour la manipulation des instruments chirurgicaux et présentant plusieurs éléments articulés entre eux en étant reliés à des moyens de commande pour être orientés à volonté.

Le brevet DE-U-8535164.4 concerne un dispositif analogue du type "pince" dans laquelle des câbles sont engagés dans des éléments articulés. Ces câbles sont montés dans une gaine.

Le brevet US-A-4 239 036 divulgue un appareil de manipulation présentant un corps cylindrique prolongé par une lame ressort.

Le problème que se propose de résoudre l'invention est d'obtenir une proportionalité entre les mouvements de l'index de l'opérateur et les mouvements des éléments d'extrémité que présente la pince. Cette proportionalité combinée avec une ergonomie spécifique, permet de travailler correctement en chirurgie coelioscopique. Notamment l'index de l'opérateur permet d'orienter correctement un instrument chirurgical susceptible d'être fixé en bout des éléments articulés. Le but recherché est d'activer les mouvements d'un instrument chirurgical tout en corrigeant la direction de son axe résultant de l'accouplement des éléments mobiles articulés entre eux.

Pour résoudre un tel problème, il a été conçu et mis au point un dispositif orientable pour la manipulation des instruments chirurgicaux, notamment en chirurgie coelioscopique. Le dispositif selon l'invention est conforme aux caractéristiques de la revendication 1.

Pour résoudre le problème posé d'assurer la protection des éléments, tout en permettant leur orientation, ces derniers sont engagés dans une gaine isolante comportant des zones de pliures destinées à faciliter la courbure de l'ensemble des éléments.

Compte-tenu du problème posé et des applications envisagées, le dispositif porte, à l'extrémité du segment, des instruments chirurgicaux, simples ou composés, fixes ou interchangeables, de taille adaptée, du type pince, bistouri, ciseaux, porte-aiguille, clampe, aiguille simple, agrafe, susceptibles d'être commandés par un organe du type gaine.

Dans le cas d'un instrument du type pince notamment, le problème posé de la manoeuvrer est résolu en ce que la gaine reçoit un câble de fonctionnement manoeuvré par un levier monté sur le corps, ladite gaine étant disposée coaxialement aux éléments en pouvant glisser librement dans l'alésage desdits éléments.

Compte-tenu des conditions de travail, notamment à une pression supérieure à la pression atmosphérique, le dispositif présente un joint d'étanchéité souple entourant la gaine et un système de tension maintenu en permanence sur la gaine par une poule dont l'axe est poussé par un ressort pour permettre à la gaine de suivre, sans les gêner, les mouvements de courbure des cylindres.

Dans une autre forme de réalisation, le dispositif comporte un corps cylindrique prolongé par une lame ressort pouvant être courbée par un câble passant dans des arceaux solidaires de la lame, ledit câble étant engagé librement dans le corps cylindrique en étant susceptible d'être bloqués par des renflements en combinaison avec une lente, l'ensemble de la lame ressort et du câble coopérant avec une gaine souple.

L'invention est exposée, ci-après plus en détail à l'aide des dessins annexés, dans lesquels :
La figure 1 est une vue en perspective des éléments du dispositif.
La figure 2 est une vue en plan correspondant à la figure 1.
La figure 3 est une vue en coupe longitudinale considérée selon la ligne 3.3 de la figure 2.
La figure 4 montre le système de guidage de la gaine.
La figure 5 montre l'ensemble du dispositif.
La figure 6 montre le moyen de commande d'orientation des éléments cylindriques.
La figure 7 est une vue en perspective d'un instrument accessoire sous forme d'un écarteur.
La figure 8 est une vue en coupe longitudinale de l'écarteur.
La figure 9 est une vue en perspective d'une gaine de forme plane.
La figure 10 montre un écarteur de forme plane.

Selon l'invention, le dispositif présente plusieurs éléments cylindriques creux et articulés entre eux (X1 à Xn). Ces éléments sont solidaires d'un corps (X0) qui présente une poignée de préhension (Q). L'articulation des éléments est déterminée pour que ce dernier se courbe à la façon d'un doigt. Le mouvement d'articulation est directement contrôlé par le doigt de l'opérateur, allant dans le même sens en utilisant un système de câbles souples (C1, C2). Le mouvement de l'extrémité de l'instrument est proportionnel à la courbure du doigt de l'opérateur.

Le dispositif décrit est conformé pour porter à son extrémité opératoire une grande variété d'instruments dont les mouvements propres sont commandés par un lever spécial. Les instruments concernés peuvent servir à la préhension, telles que pinces, clamps ou à la section, tels que ciseaux ou à l'éléctrocoagulation ou à la ponction ou à la pose de clips.

Les dessins montrent, à titre d'exemple indicatif nullement limitatif, un dispositif portant une pince de préhension.

Le dispositif est de forme générale cylindrique d'une longueur habituelle comprise entre une vingtaine et une quarantaine de centimètres selon les besoins.

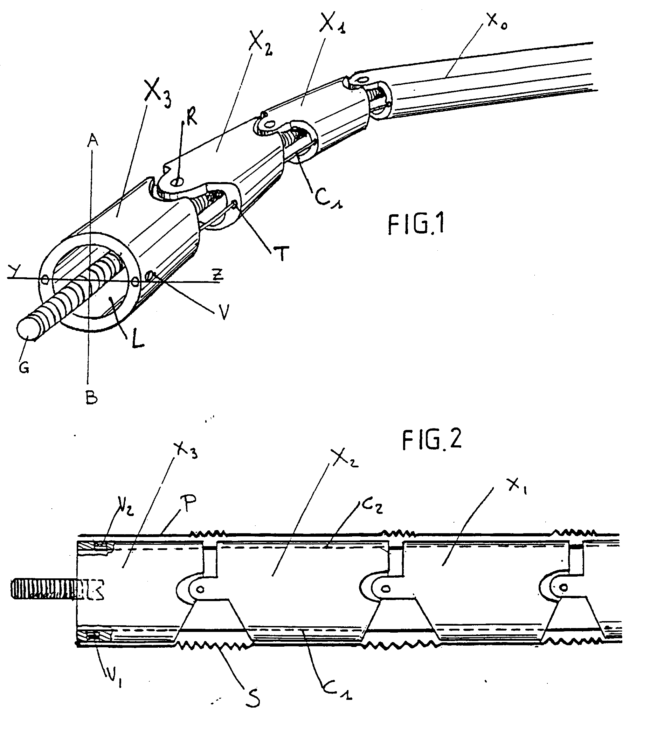

Selon l'invention, les éléments (X1, Xn) sont assujettis à des moyens de commande (C1, C2, K) pour être orientés à volonté avec capacité de blocage temporaire dans la position angulaire désirée. Ces moyens de commande sont constitués par un système de câbles (C1, C2) engagés à libre coulissement dans des passages (T) formés dans l'épaisseur de la paroi des éléments cylindriques (X1 à Xn) (figures 1, 2 et 3). Ces câbles sont asservis par une poignée de commande (K) fixée en bout du corps (X0).

Les câbles (C1 et C2) coopèrent avec un ensemble de poulies (M1, M2) présentant deux gorges et reliées à la poignée de commande (K). Cette poignée permet, par enroulement partiel des câbles (C1 et C2), de faire mouvoir les éléments (X1) à (Xn) dans un plan perpendiculaire à celui de leurs articulations et de les maintenir solidement dans la position contrôlée par la poignée de commande (K). L'extrémité libre des câbles est fixée, par exemple au moyen de vis (V1 et V2), dans l'élément situé en bout du dispositif et destiné à recevoir un instrument quelconque.

L'articulation des éléments (X1 à Xn) s'effectue par deux points d'articulation (R) diamétralement opposés sans traverser l'alésage (L) desdits éléments.

Les câbles (C1 et C2), diamétralement opposés, sont situés dans le plan (YZ), lui même perpendiculaire au plan (AB) qui donne la position des articulations (R).

Comme le montre la figure 2, l'élément d'extrémité (X3) présente une face perpendiculaire à son axe principal qui sert à porter le mouvement de l'instrument considéré. L'extrémité articulée de l'élément (X3) et l'ensemble des extrémités articulés des autres éléments comportent un plan de section formant un angle d'environ 45° par rapport à l'axe principal L'ensemble du dispositif, notamment les différents éléments (X1 à Xn) est monté dans une gaine de protection (P) entourant l'ensemble desdits éléments. Cette gaine est fixée sur les éléments et comporte des zones de pliures circulaires (S).

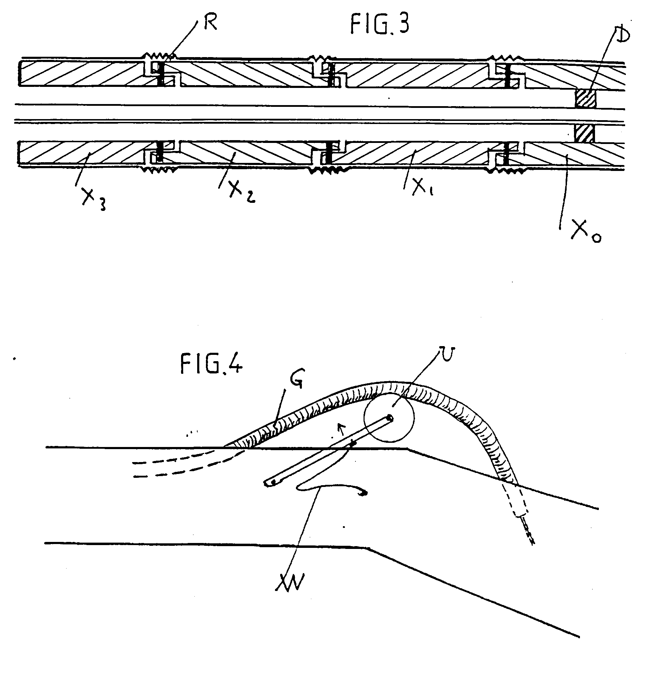

La figure 3 montre une coupe des éléments cylindriques X1, X2, X3 et X0 selon l'axe (AB) passant par les points d'articulation (R), ceux ci interessent l'épaisseur de la paroi des éléments cylindriques. Cette figure montre également l'application du dispositif dans le cas où l'instrument fixé en bout de l'élément (X3) est une pince commandée par une gaine (G). La gaine (G) est montée librement dans l'alésage des différents éléments et du corps du dispositif. Cette gaine (G) contient un câble pour la commande de la pince. Un joint (D) assure l'étanchéité.

La figure 4 montre le système de guidage de la gaine (G). Une molette (U), montée sur un bras de levier, est poussée vers le haut par un ressort (W) maintenant en permanence l'excès de longueur de la gaine (G) hors du dispositif.

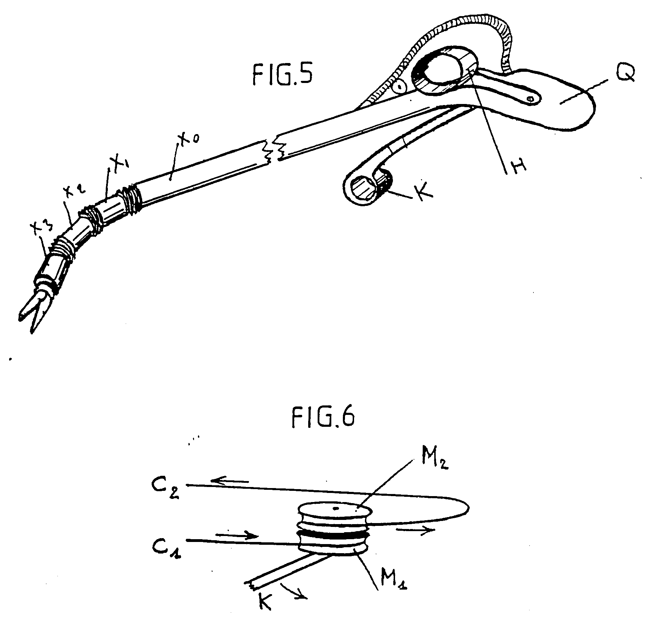

La figure 5 montre l'ensemble du dispositif comportant une poignée (Q), le lever d'orientation (K) et le lever de commande de l'instrument d'extrémité (H). Le lever (K) est assujetti aux poulies (M1, M2) pour la commande des câbles (C1, C2). On renvoie à la figure 6 qui montre le mécanisme permettant la mise en oeuvre des câbles (C1 et C2) par le lever (K) fixé sur les poulies (M1 et M2) tendant les câbles (C1 et C2).

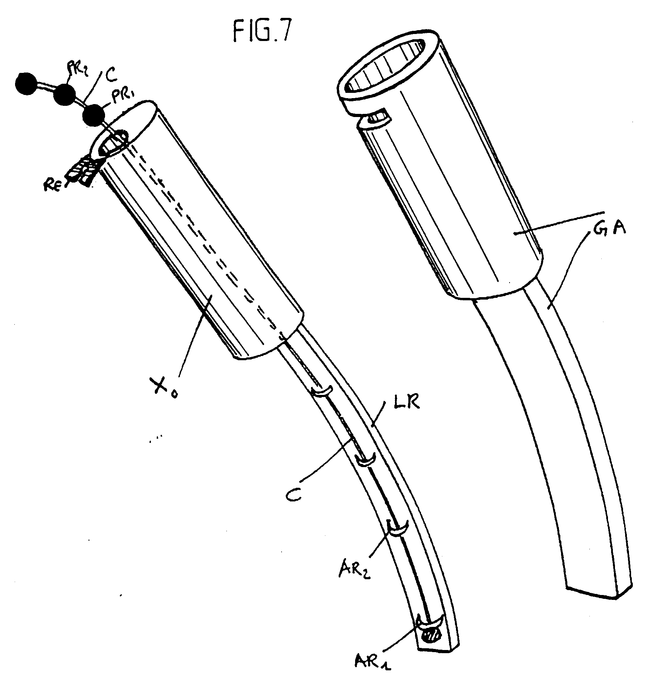

La figure 7 montre un instrument accessoire sous forme d'un écarteur comprenant un corps (X0), une lame ressort (LR) et un câble (C) fixé à l'extrémité de ladite lame. Le câble (C) passe sous des arceaux (AR1 à ARn). L'ensemble est entouré d'une gaine (GA).

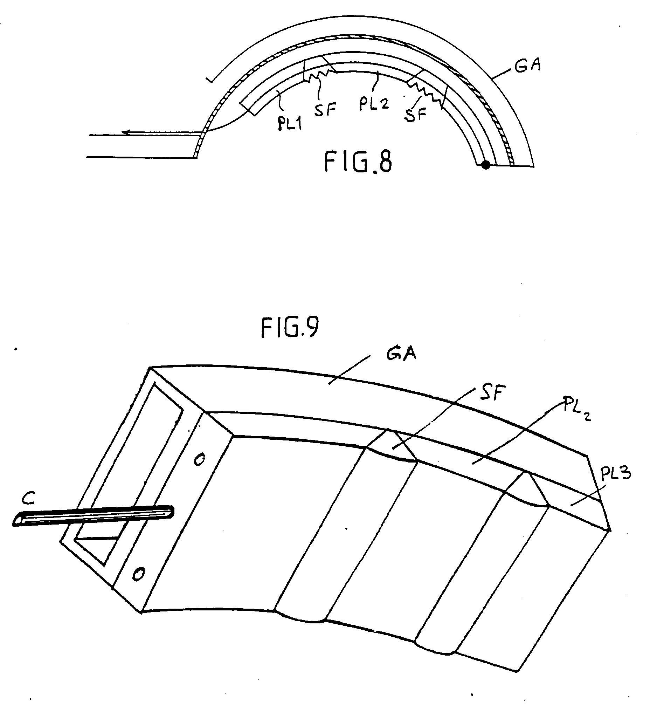

La figure 8 montre une coupe longitudinale de l'écarteur et de la gaine qui comporte plusieurs éléments (PL1 à PLn) reliés entre eux par des zones flexibles (SF).

La figure 9 montre une gaine de section plane comportant un à plusieurs orifices et canaux longitudinaux pour le passage du câble (C).

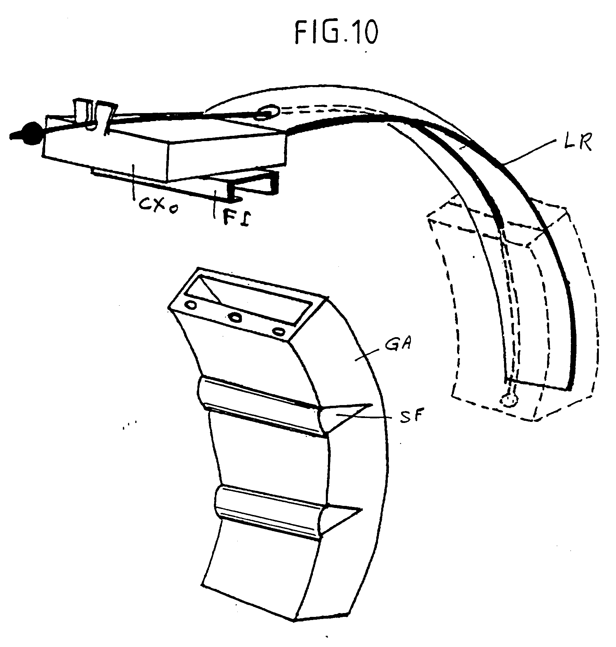

La figure 10 montre l'écarteur de forme plane comportant un corps (CX0), une lame ressort (LR) et des éléments de fixation (Fl).

La réalisation du dispositif s'effectue sur la base de plusieurs éléments cylindriques métalliques ou plastiques entourés d'une gaine de protection en matière synthétique isolante.

Le but recherché, est d'obtenir un dispositif répondant aux doigts de l'opérateur qui pourra ainsi orienter de la même main l'extrémité de l'instrument et faire manoeuvrer l'outil d'extrémité. Le travail chirurgical se faisant avec les deux mains, il sera nécessaire de construire un dispositif adapté pour chaque main, les instruments pouvant être symétriques en "miroir".

Le dispositif est tenu par sa poignée (Q). Le pouce est introduit dans la boucle du levier (H) pour actionner l'instrument d'extrémité (pince ou autre). Le levier (K) est actionné par l'index ou le majeur pour orienter les éléments cylindriques (X1, X2, X3).

Les avantages ressortent bien de la description.

## Revendications

1. Dispositif orientable pour la manipulation des instruments chirurgicaux notamment en chirurgie coelioscopique présentant plusieurs éléments cylindriques creux et articulés entre eux (X1-Xn), lesdits éléments étant solidaires d'un corps (Xo) présentant une poignée de préhension (Q), lesdits éléments (X1-Xn) étant assujettis à un système de câbles (C1,C2) engagés à libre coulissement dans l'épaisseur de la paroi des éléments (X1-Xn), pour être orientés à volonté avec capacité de blocage temporaire dans la position angulaire désirée, caractérisé en ce que les câbles (C1-C2) sont asservis par une poignée de commande (K) en combinaison avec un ensemble de poulies présentant deux gorges pour permettre par enroulement partiel desdits câbles, de faire mouvoir les éléments (X1-Xn) dans un plan perpendiculaire à celui de leurs articulations et les maintenir solidement dans la position contrôlée par la poignée de commande (K) qui est actionnée par l'index ou le majeur pour l'orientation des éléments (X1-Xn) tandis que le pouce est introduit dans une boucle d'un levier (H) pour actionner un instrument fixé à l'extrémité libre des câbles.

2. Dispositif selon la revendication 1, caractérisé en ce que les éléments (X1-Xn) sont engagés dans une gaine isolante (P) comportant des zones de pliure (O) destinées à faciliter la courbure de l'ensemble desdits éléments.

3. Dispositif selon la revendication 2, caractérisé en ce que la gaine reçoit un câble de fonctionnement manoeuvré par le levier (H), ladite gaine étant disposée coaxialement aux éléments (X1-Xn) en pouvant glisser librement dans l'alésage desdits éléments.

4. Dispositif selon la revendication 3, caractérisé en ce qu'il présente un joint souple d'étanchéité (D) entourant la gaine et un système de tension maintenu en permanence sur la gaine par une poulie dont l'axe est poussé par le ressort (W) pour permettre à la gaine de suivre sans les gêner les mouvements de courbure des éléments (X1-Xn).

5. Dispositif selon la revendication 1, caractérisé en ce qu'elle compôrte un corps cylindrique (Xo) prolongé par une lame ressort (LR) pouvant être courbée par un câble (C) passant dans des arceaux (AR1-AR2) solidaires de ladite lame, le câble (C) étant engagé librement dans le corps cylindrique (Xo) en étant susceptible d'être bloqué par des renflements (PR1-PR2) en combinaison avec une fente (RE), l'ensemble de la lame ressort (LR) et du câble (C) coopérant avec une gaine souple (GA).

6. Dispositif selon la revendication 5, caractérisé en ce que la gaine (GA) comporte plusieurs segments rattachés les uns aux autres par des zones flexibles (GF), le câble (C) étant engagé dans une partie de ladite gaine en étant libre par rapport à la lame ressort (LR).

7. Dispositif selon l'une quelconque des revendications 5 et 6, caractérisé en ce que l'extrémité de la lame (LR) est solidaire d'un corps (CXo) comportant des éléments de fixation (F1) à un écarteur autostatique et des éléments de manoeuvre du câble (C) par l'intermédiaire de renflements (PR1) à (PR2) ou d'un levier spécial.

## Claims

1. Swivel-type device for manipulating surgical instruments, especially in coelioscopic surgery, having several hollow cylindrical components (X1-Xn) flexibly joined to each other, said components being connected to a body (Xo) having a gripping handle (Q), said components (X1-Xn) being controlled by a system of wires (C1, C2) slidably fitted in the thickness of the wall of the components (X1-Xn) so that they can be oriented as required with the ability to lock them temporarily in a desired angular position, characterised in that the wires (C1-C2) are controlled by a control handle (K) in combination with an assembly of pulleys having two grooves making it possible, by partially winding said wires, to move components (X1-Xn) in a plane perpendicular to that of their articulated joints and keep them rigid in the position monitored by control handle (K) which is actuated by the index finger or the middle finger in order to orientate components (X1-Xn) whereas the thumb is placed in the ring of a lever (H) in order to operate an instrument attached to the free end of the wires.

2. Device as claimed in claim 1, characterised in that components (X1-Xn) are fitted in an insulating sheath (P) having bending areas (O) intended to facilitate curvature of all said components.

3. Device as claimed in claim 2, characterised in that the sheath accommodates an operating wire controlled by lever (H), said sheath being arranged coaxially with components (X1-Xn) and being capable of sliding freely inside the bore of said components.

4. Device as claimed in claim 3, characterised in that it has a flexible seal (D) surrounding the sheath and a tensioning system continuously held against the sheath using a pulley of which the shaft is pushed by spring (W) in order to allow the sheath to adapt to bending movements of components (X1-Xn) without impeding them.

5. Device as claimed in claim 1, characterised in that it has a cylindrical body (Xo) extending as a spring blade (LR) that can be curved by a wire (C) passing through bows (AR1-AR2) joined to said blade, wire (C) being fitted freely inside cylindrical body (Xo) and being capable of being locked by beads (PR1-PR2) in combination with a slit (RE), the spring blade (LR) and wire (C) assembly cooperating with a flexible sheath (GA).

6. Device as claimed in claim 5, characterised in that the sheath (GA) comprises several segments attached to each other by flexible areas (GF), wire (C) being fitted inside part of said sheath and being free relative to the spring blade (LR).

7. Device as claimed in any of claims 5 and 6, characterised in that the end of blade (LR) is joined to a body (CXo) comprising elements for attachment (F1) to a self-locking expander and elements for operating the wire (C) through beads (PR1) to (PR2) or a special lever.

## Patentansprüche

1. Ausrichtbare Vorrichtung für die Bedienung chirurgischer Instrumente, insbesondere in der laparoskopischen Chirurgie, mit mehreren zylindrischen und gelenkig miteinander verbundenen Hohlelementen (X1-Xn), wobei diese Elemente mit einem Körper (Xo) fest verbunden sind, der einen Griff (Q) aufweist, und von einem System aus Kabeln (C1,C2) abhängig sind, die frei gleitend in die Wandtiefe der Elemente (X1-Xn) eingeführt sind, um mit der Möglichkeit einer zeitweiligen Blockierung in der gewünschten Stellung beliebig ausgerichtet werden zu können, dadurch gekennzeichnet, daß die Kabel (C1,C2) durch einen Steuergriff (K) geführt werden, und zwar in Verbindung mit einem Seilscheibensystem, das zwei Nuten aufweist, um durch partielles Aufwickeln der Kabel die Bewegung der Elemente (X1-Xn) senkrecht zur Ebene ihrer Gelenke zu ermöglichen und sie fest in der vom Steuergriff (K) kontrollierten Position zu halten, wobei der Griff zur Ausrichtung der Elemente (X1-Xn) vom Zeigefinger oder vom Mittelfinger betätigt wird, während der Daumen in eine Schlinge eines Hebels (H) eingeführt ist, um ein am freien Ende der Kabel befestigtes Instrument zu betätigen.

2. Vorrichtung nach Anspruch 1, dadurch gekennzeichnet, daß die Elemente (X1-Xn) in eine isolierende Hülle (P) eingeführt sind, die Faltzonen (O) für das leichtere Krümmen aller Elemente besitzt.

3. Vorrichtung nach Anspruch 2, dadurch gekennzeichnet, daß die Hülle ein Funktionskabel aufnimmt, das von dem Hebel (H) betätigt wird, wobei die Hülle koaxial zu den Elementen (X1-Xn) verläuft und in der Bohrung der Elemente unbehindert gleiten kann.

4. Vorrichtung nach Anspruch 3, dadurch gekennzeichnet, daß die Hülle von einem elastischen Dichtelement (D) umgeben wird und daß ein Spannungssystem durch eine Seilscheibe, deren Achse von der Feder (W) vorgedrückt wird, ständig auf der Hülle gehalten wird, damit diese den Krümmungsbewegungen der Elemente (X1-Xn) folgen kann, ohne sie zu behindern.

5. Vorrichtung nach Anspruch 1, dadurch gekennzeichnet, daß sie einen zylindrischen Körper (Xo) besitzt, der durch ein Federblatt (LR) verlängert wird, das durch ein Kabel (C) gekrümmt werden kann, welches durch mit dem Blatt fest verbundene Bögen (AR1-AR2) verläuft, wobei das Kabel (C) unbehindert in den zylindrischen Körper (Xo) eingeführt ist und dabei in Verbindung mit einem Schlitz (RE) durch Verdickungen (PR1-PR2) blockiert werden kann und das System aus Federblatt (LR) und Kabel (C) mit einer elastischen Hülle (GA) zusammenwirkt.

6. Vorrichtung nach Anspruch 5, dadurch gekennzeichnet, daß die Hülle (GA) mehrere Segmente aufweist, die durch flexible Zonen (GF) miteinander verbunden sind, wobei das Kabel (C) in einen Teil der Hülle eingeführt und gegenüber dem Federblatt (LR) frei beweglich ist.

7. Vorrichtung nach Anspruch 5 oder 6, dadurch gekennzeichnet, daß das Ende des Blattes (LR) mit einem Körper (CXo) fest verbunden ist, der Elemente (F1) zur Befestigung an einem eigenstatischen Abstandshalter und Elemente für die Betätigung des Kabel (C) durch Verdickungen (PR1)-(PR2) oder einen Spezialhebel besitzt.
